# EUROPEAN PATENT APPLICATION

(11) **EP 0 853 941 A2**
(43) Date of publication of application: **22.07.1998**
(21) Application number: 97121657.7
(22) Date of filing: 09.12.1997
(51) Int. Cl.: A61K 7/50

(54) **Detergent composition**

(30) Priority: 20.12.1996 JP 341657/96; 27.12.1996 JP 349933/96
(71) Applicant: Kao Corporation, Chuo-Ku Tokyo (JP)
(72) Inventor: Sonohara, Hiroshi, c/o Kao Corporation, Wakayama-shi, Wakayama (JP); Hasebe, Keiko, c/o Kao Corporation, Wakayama-shi, Wakayama (JP); Shonaka, Masafumi, c/o Kao Corporation, Wakayama-shi, Wakayama (JP); Doi, Yasuhiro, c/o Kao Corporation, Wakayama-shi, Wakayama (JP)
(74) Representative: Hansen, Bernd, Dr. Dipl.-Chem.

(57) **Abstract**

Disclosed herein is a detergent composition comprising (A) 5-95 wt.% of a higher fatty acid salt and/or a sulfuric ester type surfactant, (B) 0.2-5 wt.% of a cationic germicide, (C) 0.1-10 wt.% of at least one selected from the group consisting of amphoteric surfactants, amine oxide type surfactants, alkanolamide type surfactants and amidoamino acid type surfactants, and (D) 0.01-5 wt.% of a cationic polymer, wherein the component (A) is contained as a principal detergent base. The detergent composition has excellent detergency and foamability, gives users a pleasant feeling after use, and has a high germicidal effect because a cationic germicide can be stably incorporated therein.

## Description

### BACKGROUND OF THE INVENTION

### Field of the Invention:

The present invention relates to detergent compositions, and particularly to detergent compositions which have low irritativeness, and excellent detergency and foamability, give users a pleasant feeling after use, further have excellent antipruritic and deodorant effects on the body and scalp and excellent an antidandruffy effect on the scalp because a cationic germicide can be stably incorporated therein to exhibit high germicidal and antibacterial effects, and are suitable for, in particular, skin and hair detergents.

### Description of the Background Art:

Higher fatty acid salts as anionic surfactants have heretofore been often used as main base materials for detergents, in particular, skin detergents because they have excellent detergency and foamability. However, the higher fatty acid salts have such drawbacks that they have high irritativeness to the skin, and cause skin roughness and skin stiffness after use when they are used singly. Besides, anionic surfactants such as polyoxyethylene alkyl ether sulfates or alkenyl ether sulfates have been often used as main base materials for hair shampoos with a view toward achieving excellent detergency and foamability.

On the other hand, detergents, particularly, hair and body shampoos, are also required to have germicidal and antibacterial effects in addition to the detergent effect. Many investigations have been made as to detergents having a germicidal effect with a view toward preventing formation of dandruff and itch, and detergent compositions making use of a cationic germicide as a germicide have been known.

However, the combined use of an anionic surfactant with a cationic germicide has involved a problem that they form a complex, so that not only the detergency is lowered, but also the germicidal effect cannot be sufficiently exhibited.

As methods for preventing such reduction in germicidal activity, there have been used a method in which the cationic germicide is coated with a high molecular weight nonionic surfactant or a pH-sensitive polymer, and a method in which the cationic germicide is added in excess. However, the former polymer coating method reduces the activity of the cationic germicide. On the other hand, the method making use of the germicide in excess is not preferable from an economical point of view.

### SUMMARY OF THE INVENTION

Accordingly, it is an object of the present invention to provide detergent compositions which have excellent detergency and foamability, give users a pleasant feeling and no dry and stiff feeling toward the skin after use, have low irritativeness, further have excellent antipruritic and deodorant effects on the body and scalp and excellent antidandruffy effects on the scalp because a cationic germicide can be stably incorporated therein to bring about high germicidal and antibacterial effects, and are hence suitable for skin and hair detergents.

In view of the foregoing circumstances, the present inventors have carried out an extensive investigation with a view toward solving the above-described problems. As a result, it has been found that when a higher fatty acid salt or a sulfuric ester type surfactant, a specific cationic germicide, at least one selected from the group consisting of amphoteric surfactants, amine oxide type surfactants, alkanolamide type surfactants and amidoamino acid type surfactants, and a cationic polymer are incorporated in combination in specific proportions, a detergent composition which has high detergency and foamability and low irritativeness, gives users a pleasant feeling after use, has satisfactory germicidal, antibacterial, antipruritic, deodorant and antidandruffy effects on the body and scalp and excellent effect, and is suitable for a skin or hair detergent can be provided, thus leading to completion of the present invention.

According to the present invention, there is thus provided a detergent composition comprising the following components (A), (B), (C) and (D):
(A) 5-95 wt.% of at least one anionic surfactant selected from the group consisting of higher fatty acid salts represented by the general formula (1):

   R¹COOM¹ (1)

   wherein R¹ is a linear or branched alkyl or alkenyl group having 7-21 carbon atoms, and M¹ is an alkali metal or alkaline earth metal atom, or an ammonium, alkylammonium or alkanolammonium group, and sulfuric ester type surfactants represented by the general formula (2):

   R²O-(CH₂CH₂O)ₙ-SO₃M² (2)

   wherein R² is a linear or branched alkyl or alkenyl group having 8-20 carbon atoms, n is a number of 0-10 on the average, and M² is an alkali metal or alkaline earth metal atom, or an ammonium, alkylammonium or alkanolammonium group;
(B) 0.2-5 wt.% of at least one selected from the group consisting of cationic germicides represented by the following general formulae (3), (4), (5) and (6): 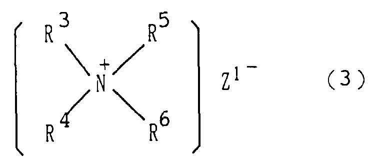 wherein R³ and R⁴ may be the same or different from each other and are independently a long-chain alkyl, long-chain alkenyl or long-chain hydroxyalkyl group having 6-14 carbon atoms, said groups R³ and R⁴ having 16-26 carbon atoms in total, R⁵ and R⁶ may be the same or different from each other and are independently an alkyl or hydroxyalkyl group having 1-3 carbon atoms, or a polyoxyethylene group having an average number of moles of at most 10, and Z¹ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin; 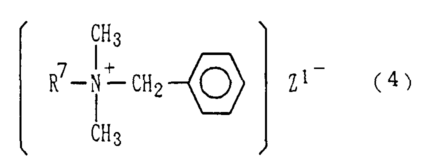 wherein R⁷ is a hydrocarbon group having 8-14 carbon atoms or a group represented by the formula: 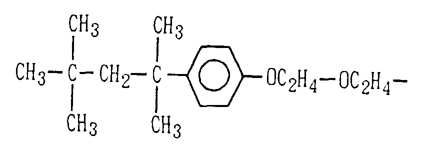 and Z¹ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerisation degree of at least 3 or containing a condensate of a sulfonated polycyclic aromatic compound, which may have a hydrocarbon group as a substituent group, with formalin; 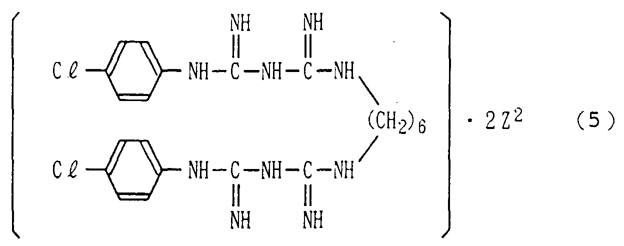 wherein Z² is gluconic acid, acetic acid or hydrochloric acid; and 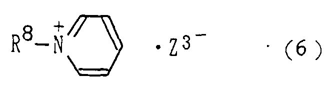 wherein R⁸ is a linear or branched alkyl group having 6-18 carbon atoms, Z³ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin, and polyhexamethylene biguanide;
(C) 0.1-10 wt.% of at least one selected from the group consisting of amphoteric surfactants, amine oxide type surfactants, alkanolamide type surfactants and amidoamino acid type surfactants; and
(D) 0.01-5 wt.% of a cationic polymer, wherein the component (A) is contained as a principal detergent base.
   The detergent compositions according to the present invention have excellent detergency and foamability, give users a pleasant feeling after use, and further have sufficient antidandruffy effects on the scalp and sufficient antipruritic and deodorant effects on the body and scalp because a cationic germicide can be stably incorporated therein to exhibit high germicidal and antibacterial effects and broad germicidal and antibacterial spectra.

The above and other objects, features and advantages of the present invention will be readily appreciated as the same becomes better understood from the preferred embodiments of the present invention, which will be described subsequently in detail, and from the appended claims.

### DETAILED DESCRIPTION OF THE PREFERRED EMBODIMENTS

Specific examples of the higher fatty acid salt of the component (A) useful in the practice of the present invention include alkali metal salts, alkaline earth metal salts, ammonium salts, alkylammonium salts and alkanolamine salts of caprylic acid, capric acid, undecanoic acid, lauric acid, tridecanoic acid, myristic acid, pentadecanoic acid, palmitic acid, heptadecanoic acid, stearic acid, oleic acid, elaidic acid, linoleic acid, linolenic acid, arachidic acid, and fatty acids derived from animal and vegetable fats and oils, such as coconut oil fatty acid and beef tallow fatty acid containing these fatty acids. Preferable examples thereof include those in which R¹ in the general formula (1) is a linear or branched alkyl or alkenyl group having 11-17 carbon atoms.

It is preferable that M¹ in the general formula (1) be suitably varied according to the form of the detergent composition according to the present invention. Specifically, when the detergent composition is used in the form of, for example, liquid or paste, M¹ is preferably a potassium atom, or an ammonium or triethanolammonium group. When the composition is used in the form of solid, M¹ is preferably a sodium atom.

In the general formula (2) which represents the sulfuric ester type surfactant of the component (A) useful in the practice of the present invention, R² is preferably a linear or branched alkyl or alkenyl group having 10-16 carbon atoms, and n is preferably a number of 0-6 on the average from the viewpoint of detergency and foamability. n is more preferably a number of 0-4, with 1-4 being particularly preferred. M² is particularly preferably a sodium atom, or an ammonium or triethanolammonium group.

The component (A) is incorporated as a principal detergent base for the detergent compositions according to the present invention, and the above-described anionic surfactants may be used either singly or in any combination thereof in the detergent compositions. The component (A) is incorporated in a proportion of at least 50 wt.% (hereinafter indicated merely by "%") based on the total weight of the detergent surfactants.

The component (A) is preferably incorporated in a varied amount according to the form of the detergent composition according to the present invention. Specifically, when the detergent composition is provided in the form of, for example, liquid, the component (A) is preferably incorporated in a proportion of 5-50%, particularly, 10-40% based on the total weight of the composition. When the composition is provided in the form of paste, the component (A) is preferably incorporated in a proportion of 10-80%, particularly, 15-40% based on the total weight of the composition. When the composition is provided in the form of solid, the component (A) is preferably incorporated in a proportion of generally 60-95%, particularly, 70-95% based on the total weight of the composition.

When the detergent composition according to the present invention is provided as a skin detergent, it is preferable that the higher fatty acid salt be used as a principal detergent base, and the sulfuric ester type surfactant be used in combination with the higher fatty acid salt if desired. When the detergent composition is provided as a hair shampoo on the other hand, it is preferable that the sulfuric ester type surfactant be used as a principal detergent base, and the higher fatty acid salt be used in combination with the sulfuric ester type surfactant if desired.

The cationic germicide of the component (B) useful in the practice of the present invention is at least one selected from the group consisting of quaternary ammonium salts represented by the general formula (3), benzalkonium salts or benzethonium salts represented by the general formula (4), chlorhexidine salts represented by the general formula (5), pyridinium salts represented by the general formula (6) and polyhexamethylene biguanide.

Here, polyhexamethylene biguanide corresponds to that described as POLYAMINOPROPYL BIGUANIDE in International Cosmetic Ingredient Dictionary, the fifth edition, of CTFA, and is known by the trade mark of "Cosmocil" or "Mikrokill".

In the general formulae (3) and (4), Z¹ is particularly preferably a halogen atom.

Specific preferable examples of the component (B) include benzalkonium chloride, benzethonium chloride, cetyl pyridinium chloride, chlorhexidine gluconate, chlorhexidine acetate and chlorhexidine hydrochloride. Of these, benzalkonium chloride and benzethonium chloride are particularly preferred. Further, benzalkonium chloride represented by the general formula (4a); 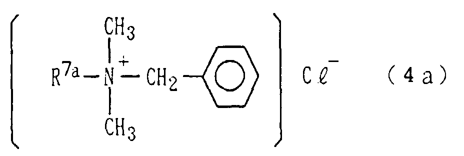 wherein R^{7a} is a linear or branched alkyl or alkenyl group having 8-14 carbon atoms, is preferred.

The cationic germicides of the component (B) may be used either singly or in any combination thereof, and are incorporated in a proportion of 0.2-5%, preferably 0.3-4%, particularly 0.5-2% based on the total weight of the composition from the viewpoints of germicidal and antibacterial effects and irritativeness.

Examples of the amphoteric surfactants of the component (C) useful in the practice of the present invention include betaine type amphoteric surfactants represented by the following general formula (7): 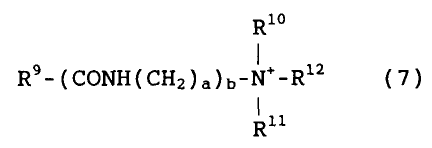 wherein R⁹ is a linear or branched alkyl or alkenyl group having 7-22 carbon atoms, a is an integer of 1-4, b is a number of 0 or 1, R¹⁰ and R¹¹ are independently an alkyl group having 1-4 carbon atoms or -(CH₂CH₂O)_{d}H, in which d is a number of 1-3 on the average, and R¹² is -CH₂CH(OH)CH₂SO₃⁻, -(CH₂)ₑSO₃⁻ or -(CH₂)_{f}COO⁻, in which e is an integer of 2-5, and f is an integer of 1-3.

Of these, preferred are carbobetaine type surfactants represented by the general formula (7a): 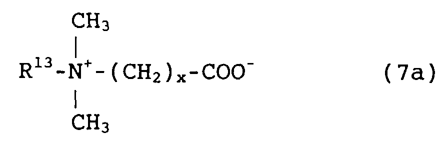 wherein R¹³ is a linear or branched alkyl or alkenyl group having 8-20 carbon atoms or a group represented by R¹⁴CONH(CH₂)_{y}-, in which R¹⁴ is a linear or branched alkyl or alkenyl group having 7-19 carbon atoms, and y is an integer of 1-5, and x is an integer of 1-5; and sulfobetaine or hydroxysulfobetaine type surfactants represented by the general formula (7b) or (7c): 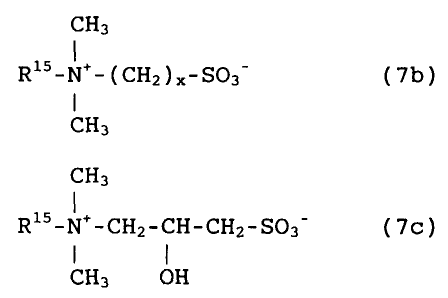 wherein R¹⁵ is a linear or branched alkyl or alkenyl group having 8-20 carbon atoms, and x has the same meaning as defined above. In the general formula (7a), R¹³ is preferably a linear or branched alkyl or alkenyl group having 8-16 carbon atoms or R¹⁴CONH(CH₂)₃-, in which R¹⁴ is a linear or branched alkyl or alkenyl group having 7-15 carbon atoms, and x is preferably 1. In the general formula (7b) or (7c), R¹⁵ is preferably a linear or branched alkyl or alkenyl group having 8-16 carbon atoms. Further, fatty acid amide propylbetaines, wherein R¹³ in the general formula (7a) is R¹⁴CONH(CH₂)_{y}-, in which R¹⁴ is a linear or branched alkyl or alkenyl group having 7-15 carbon atoms, and y is 3, are particularly preferred.

Examples of the amine oxide type surfactants of the component (C) useful in the practice of the present invention include those represented by the general formula (8): 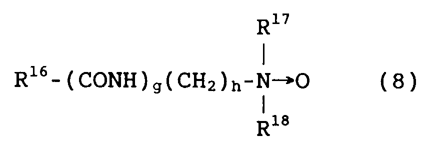 wherein R¹⁶ is a linear or branched alkyl or alkenyl group having 7-18 carbon atoms, R¹⁷ and R¹⁸ are independently a methyl, ethyl or hydroxyethyl group, g is 0 or 1, and h is an integer of 0-3. Of these, those in which R¹⁷ and R¹⁸, in the general formula (8) are both methyl groups, and g and h are both 0, are preferred.

Examples of the alkanolamide type surfactants of the component (C) useful in the practice of the present invention include those represented by the general formula (9): 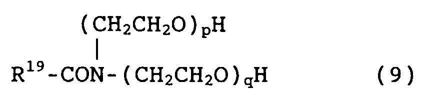 wherein R¹⁹ is a linear or branched alkyl or alkenyl group having 7-19 carbon atoms, and p and q are independently an integer of 0-10, with the proviso that at least one of p and q is 1 or greater. Of these, those, in which R¹⁹ in the general formula (9) is a linear or branched alkyl or alkenyl group having 7-15 carbon atoms, and p + q is 1-5, particularly 1 or 2, are preferred.

Examples of the amidoamino acid type surfactants of the component (C) useful in the practice of the present invention include those represented by the general formula (10) or (11): 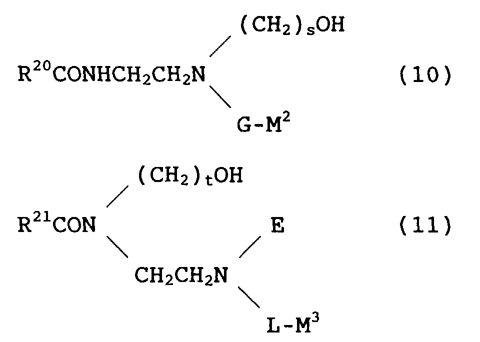 wherein R²⁰ and R²¹ are independently a linear or branched alkyl or alkenyl group having 7-19 carbon atoms, s and t are independently an integer of 2-4, G and L are independently -CH₂CH(OH)CH₂SO₃, -(CH₂)ᵤSO₃ or -(CH₂)ᵥCOO, in which u is an integer of 2-5, and v is an integer of 1-3, E is a hydrogen atom or -L-M³, and M² and M³ are independently a hydrogen atom, an alkali metal or alkaline earth metal atom, or an ammonium, organic ammonium or a mixture thereof.

Specific examples of M² and M³ include hydrogen, lithium, sodium, potassium, 1/2 calcium, 1/2 magnesium, ammonium, monoethanolammonium, diethanolammonium, triethanolammonium, monoisopropanolammonium, diisopropanolammonium and triisopropanolammonium.

The amidoamino acid type surfactants are surfactants obtained, for example, by reacting 1 mol of an imidazoline derivative with 1-2 mol of monochloroacetic acid or an acrylic acid, have once called imidazoline type amphoteric surfactants or imidazolinium betaines, correspond to those described as SODIUM COCOAMPHOACETATE, SODIUM COCOAMPHOPROPIONATE, SODIUM COCOAMPHOHYDROXYPROPYLSULFONATE, SODIUM LAUROAMPHOACETATE and DISODIUM COCOAMPHODIACETATE in the CTFA's Dictionary, the fifth edition, and are known by the trade mark of "Miranol".

Said at least one surfactant of the component (C) selected from the group consisting of the amphoteric surfactants, amine oxide type surfactants, alkanolamide type surfactants and amidoamino acid type surfactants is incorporated in a proportion of 0.1-10%, preferably 1-8%, particularly preferably 2-7% based on the total weight of the composition from the viewpoints of detergency, foamability, germicidal and antibacterial effects, and irritativeness

The weight ratio of the component (A) to the component (C) in the detergent compositions according to the present invention is preferably 1:1 to 50:1, more preferably 1:1 to 20:1, most preferably 2:1 to 15:1 from the viewpoint of the germicidal effect.

Examples of the cationic polymer of the component (D) useful in the practice of the present invention include cationic cellulose derivatives, cationic starch, diallyl quaternary ammonium salt-acrylamide copolymers, quaternized poly(vinyl pyrrolidone) derivatives, quaternized vinylpyrrolidone-vinylimidazole polymers (for example, LUVIQUAT, product of BASF AG), polyglycol polyamine condensates, quaternized collagen polypeptides, polyethyleneimine, cationic silicone polymers [for example, AMODIMETHICONE described in CTFA, product of Dow Chemical Co., a cationic silicone polymer sold as a mixture with other components under the name of Dow Corning 929 (cationic emulsion)], adipic acid-dimethylaminohydroxy-propyldiethylenetriamine copolymers (for example, Cartaretin, product of Sandoz Co. in America), polyaminopolyamide (for example, polymers described in French Patent No. 2,252,840, and crosslinked water-soluble polymers thereof), cationic chitin, cationized guar gum derivatives (for example, Jaguar C-13·S, Jaguar C-17 and Jaguar C-16, products of Celanese Corp.), quaternary ammonium salt polymers (for example, Mirapol A-15, Mirapol AD-1 and Mirapol AZ-1, products of Rhone-Poulenc in America; and polymers described in U.S. Patent Nos. 2,261,002, 2,271,378, 2,273,780, 2,388,614, 2,454,547 and 3,206,462), and cationic polymers selected from the group consisting of the following polymers (a), (b) and (c).
(a) Polymers obtained by reacting polyalkylene-polyamine having two primary amino groups and at least one secondary amino group with a dicarboxylic acid selected from among diglycolic acid and saturated aliphatic dicarboxylic acids having 3-8 carbon atoms at a molar ratio of the polyalkylenepolyamine to the dicarboxylic acid of 0.8:1 to 1.4:1, and then reacting the polyaminopolyamide formed with epichlorohydrin at a molar ratio of the epichlorohydrin to the secondary amino group of the polyaminopolyamide of 0.5:1 to 1.8:1. The polymers are described in U.S. Patent Nos. 3,337,615 and 2,961,347.
   The polymer of this type is particularly that sold under the name of Hercoset 57 by Hercules Inc., and a 10% aqueous solution thereof has a viscosity of 30 cP at 25°C. In the case of an epoxypropyl adipate-diethylenetriamine copolymer, it is sold under the name of PD170 or Delset 101 by the same company.
(b) Homopolymers or copolymers derived from acrylic acid or methacrylic acid, which have the following unit: 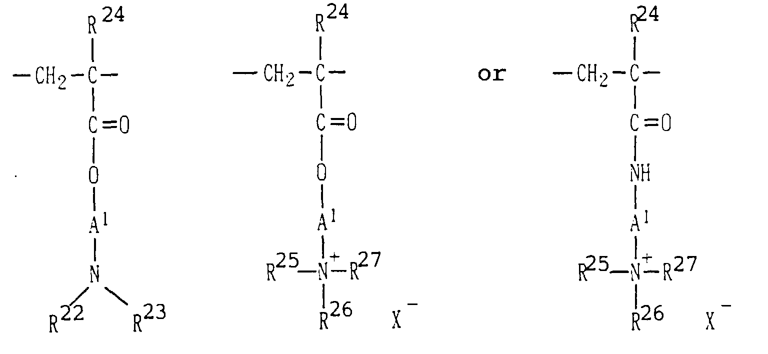 wherein R²⁴ is a hydrogen atom or a methyl group, A¹ is a linear or branched alkyl group having 1-6 carbon atom or a hydroxyalkyl group having 1-4 carbon atoms, R²⁵ R²⁶ and R²⁷ are the same or different from one another and are independently an alkyl group having 1-18 carbon atoms or a benzyl group, R²² and R²³ are independently a hydrogen atom or an alkyl group having 1-6 carbon atom, and X¹ is a methosulfate anion, or a halide such as chloride or bromide:
   Examples of monomers or monomer mixtures usable in the above copolymers include methacrylamide, diacetoneacrylamide, acrylamide and methacrylamide on the nitrogen of which a lower alkyl group is substituted, alkyl esters of acrylic acid and methacrylic acid, vinylpyrrolidone and vinyl esters.
   Specific examples of the homopolymers or copolymers include:
   products described under the names of QUATERNIUM 38, 37, 49 and 42 in Cosmetic Ingredient Dictionary;
   acrylamide/β-methacryloyloxyethyltrimethylammonium methosulfate copolymers sold under the names of Reten 205, 210, 220 and 240 by Hercules Inc.;
   aminoethyl acrylate phosphate/acrylate copolymer sold under the name of Catrex by National Starch Co., the 18% aqueous solution of which has a viscosity of 700 cP at 25°C; and
   crosslinked cationic graft copolymers having a molecular weight of 10,000 to 1,000,000, preferably 15,000 to 500,000 and obtained by copolymerization of:
      a) at least one monomer for cosmetics;
      b) dimethylaminoethyl methacrylate; c) polyethylene glycol; and
      d) poly-unsaturated crosslinking agent.
      These copolymers are described in French Patent No. 2,189,434.

   The crosslinking agent is selected from the group consisting of ethylene glycol dimethacrylate, diallyl phthalate, divinylbenzene, tetraallyloxyethane and polyallylsucrose having 2-5 allyl groups per mole of sucrose.
   The monomer for cosmetics extends over a very wide range of types, and examples thereof include vinyl esters of carboxylic acids having 2-18 carbon atoms, allyl or methallyl esters of carboxylic acids having 2-18 carbon atoms, acrylates or methacrylates of saturated alcohols having 1-18 carbon atoms, alkyl vinyl ethers the alkyl groups of which have 2-18 carbon atoms, olefins having 4-18 carbon atoms, vinyl heterocyclic derivatives, dialkyl or N,N-dialkylaminoalkyl maleates the alkyl groups of which have 1-3 carbon atoms, and unsaturated acid anhydrides.
(c) Cationic polymers selected from the group consisting of polymers having a unit represented by the formula (I):

   -A²-Z⁴-A²-Z⁴- (I)

   wherein A² is a group having two amino groups, preferably, piperazyl groups, and Z⁴ denotes a symbol B or B', in which B and B' are the same or different from each other and are independently a linear or branched alkylene group which may or may not be substituted by a hydroxyl group and may have an oxygen, nitrogen and/or sulfur atom, and 1 to 3 aromatic rings and/or heterocyclic rings; and polymers having a unit represented by the formula (II):

   -A²-Z⁵-A²-Z⁵- (II)

   wherein A² has the same meaning as defined above, and Z⁵ denotes a symbol B¹ or B^{1'}, in which at least one Z⁵ is a B^{1'}, B¹ is a linear or branched alkylene or hydroxyalkylene group, and B^{1'} is a linear or branched alkylene group which may or may not be substituted by at least one hydroxyl group and has at least one nitrogen atom in its chain, on said nitrogen atom, an alkyl group optionally having an oxygen atom in its chain and optionally having at least one hydroxyl group being substituted; as well as quaternary ammonium salts and oxidized products of the polymers of the formulae (I) and (II).

Of such various cationic polymers as described above, the cationic cellulose derivatives, cationic starch, cationized guar gum derivatives, diallyl quaternary ammonium salt-acrylamide copolymers, quaternized poly(vinyl pyrrolidone) derivatives and polyglycol polyamine condensates are particularly preferred.

As the cationic cellulose derivatives, are preferred, for examples, those represented by the following general formula (III): 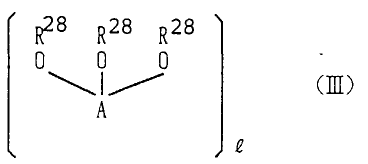 wherein A is a residue of an anhydroglucose unit, l is an integer of 50-20,000, and each R²⁸ is independently a substituent group represented by the following general formula (IV): 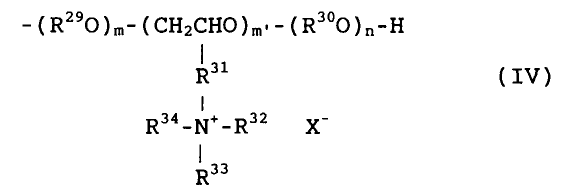 in which R²⁹ and R³⁰ are independently an alkylene group having 2-3 carbon atoms, m is an integer of 0-10, m' is an integer of 0-3, n is an integer of 0-10, R³¹ is an alkylene or hydroxyalkylene group having 1-3 carbon atoms, R³², R³³ and R³⁴ are the same or different from one another and are independently an alkyl, aryl or aralkyl group having up to 10 carbon atom, or may form a heterocyclic ring together with the nitrogen atom contained in the formula, and X is an anion (chloride, bromide, iodide, sulfate, sulfonate, methylsulfate, phosphate, nitrate or the like).

The degree of substitution of the cations in these cationic cellulose derivatives is preferably 0.01-1, namely, the average value of m' per anhydroglucose unit is preferably 0.01-1, particularly 0.02-0.5. The sum of m and n is 1-3 on the average. Any degree of substitution of the cations lower than 0.01 is insufficient. The degree of substitution may exceed 1. However, it is preferably not higher than 1 from the viewpoint of yield in the reaction. The molecular weight of the cationic cellulose derivative used herein is within a range of about 100,000-3,000,000.

As the cationic starch, are preferred those represented by the following general formula (V): 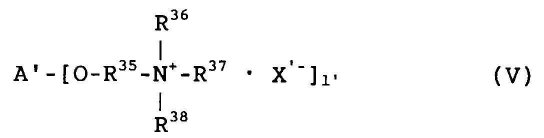 wherein A' is a residue of starch, R³⁵ is an alkylene or hydroxyalkylene group, R³⁶, R³⁷ and R³⁸ are the same or different from one another and are independently an alkyl, aryl or aralkyl group having at most 10 carbon atoms or may form a heterocyclic ring together with the nitrogen atom contained in the formula, X' is an anion such as chloride, bromide, iodide, sulfate, sulfonate, methylsulfate, phosphate or nitrate, and 1' is a positive integer.

The degree of substitution of the cations in these cationic starch derivatives is preferably 0.01-1, namely, the cationic groups are preferably introduced in a proportion of 0.01-1 group, particularly 0.02-0.5 groups per anhydroglucose unit. Any degree of substitution of the cations lower than 0.01 is insufficient. The degree of substitution may exceed 1. However, it is preferably not higher than 1 from the viewpoint of yield in the reaction.

As the cationic diallyl quaternary ammonium salt-acrylamide copolymers, are preferred those represented by the following general formula (VI) or (VII): 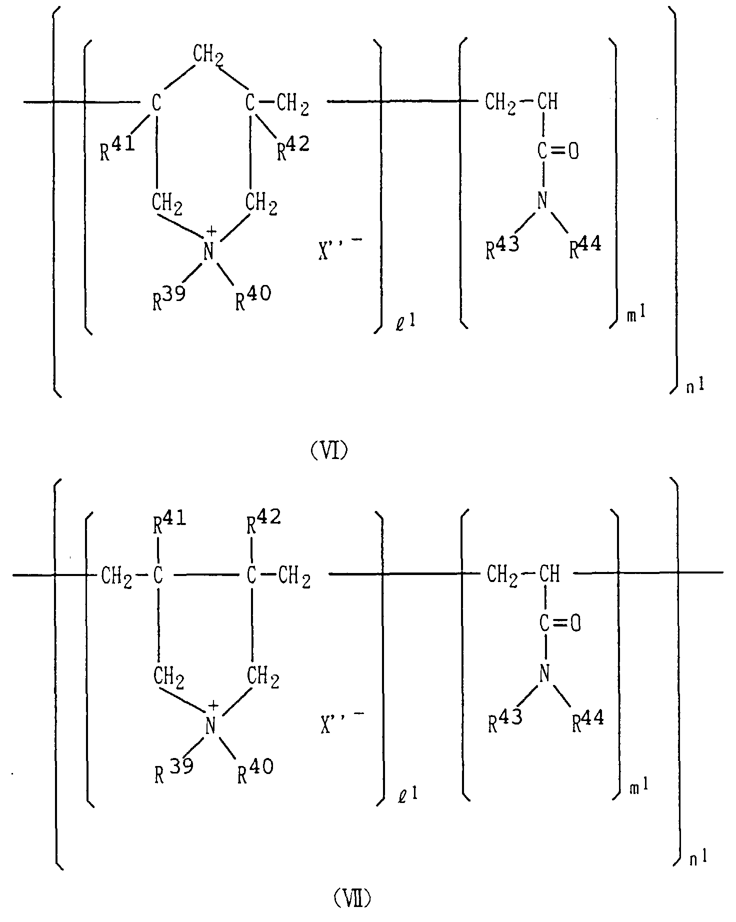 wherein R³⁹ and R⁴⁰ are the same or different from each other and are independently a hydrogen atom, or an alkyl (having 1-18 carbon atoms), phenyl, aryl, hydroxyalkyl, amidoalkyl, cyanoalkyl, alkoxyalkyl or carboalkoxyalkyl group, R⁴¹, R⁴², R⁴³ and R⁴⁴ are the same or different from one another and are independently a hydrogen atom, or a lower alkyl (having 1-3 carbon atoms) or phenyl group, X'' is an anion (chloride, bromide, iodide, sulfate, sulfonate, methylsulfate, phosphate, nitrate or the like), l¹ is an integer of 1-50, m¹ is an integer of 0-50, and n¹ is an integer of 150-8,000.

These diallyl quaternary ammonium salt-acrylamide copolymers preferably have a molecular weight within a range of about 30,000-2,000,000, particularly 100,000-1,000,000.

As the quaternized poly(vinyl pyrrolidone) derivatives, are preferred those represented by the following general formula (VIII): 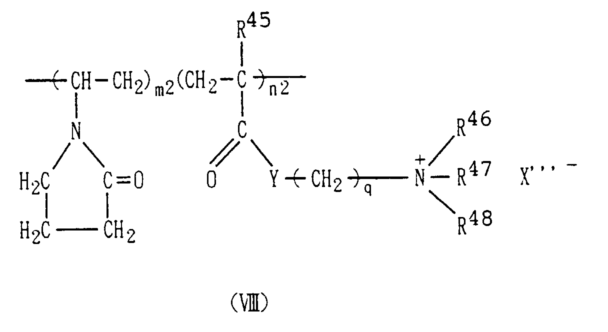 wherein R⁴⁵ is a hydrogen atom or an alkyl group having 1-3 carbon atoms, R⁴⁶, R⁴⁷ and R⁴⁸ are the same or different from one another and are independently a hydrogen atom, or an alkyl, hydroxyalkyl, amidoalkyl, cyanoalkyl, alkoxyalkyl or carboalkoxyalkyl having 1-4 carbon atoms, Y is an oxygen atom or an NH group in an amide bond, X''' is an anion such as chloride, bromide, iodide, sulfate, sulfonate, alkylsulfate having 1-4 carbon atoms, phosphate or nitrate, v is an integer of 1-10, and m² and n² are such numbers that m² plus n² is 20 to 8,000.

These quaternized poly(vinyl pyrrolidone) derivatives preferably have a molecular weight within a range of 10,000-2,000,000, particularly 50,000-1,500,000.

The content of the cationic nitrogen contained in the vinyl polymers and derived from the cationic polymer is 0.004-0.2%, preferably 0.01-0.15% based on the vinyl polymer. If the content is lower than 0.004%, a sufficient effect cannot be achieved. Any content exceeding 0.2% may be preferable from the viewpoint of performance, but forms the cause that the vinyl polymer is colored, and is economically disadvantageous.

As the polyglycol polyamine condensates, are preferred those represented by the following general formula (IX): 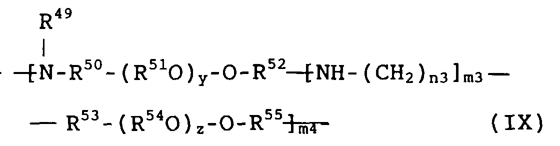 wherein R⁵⁰, R⁵², R⁵³ and R⁵⁵ are independently a hydroxyalkylene group having 2-4 carbon atoms, R⁵¹ and R⁵⁴ are independently an alkylene group having 2-3 carbon atoms, y and z are independently an integer of 10 to 20, m³ is an integer of 2 to 4, n³ is an integer of 2 to 6, m⁴ is an integer of 1 to 50, and R⁴⁹ is a linear or branched alkyl group having 6-20 carbon atoms.

As the component (D), one or more of the above-described cationic polymers are used either singly or in any combination thereof, and are preferably incorporated in a proportion of 0.01-5%, particularly 0.1-2% based on the total weight of the composition.

In the detergent compositions according to the present invention, a metal chelating agent may be incorporated as a component (E) to more enhance the germicidal and antibacterial effects of the compositions. No particular limitation is imposed on such a metal chelating agent of the component (E) so far as it has a capacity to chelate metal ions. However, examples thereof include aminopolycarboxylic acid type chelating agents, aromatic and aliphatic carboxylic acid type chelating agents, amino acid type chelating agents, ether polycarboxylic acid type chelating agents, phosphonic acid type chelating agents such as iminodimethylphosphonic acid (IDP), alkyldiphosphonic acids (ADPAs) and 1-hydroxy-ethane-1,1-diphosphonic acid (DEQUEST™ 2010), hydroxycarboxylic acid type chelating agents, phosphoric acid type chelating agents, chelating agents of the polyelectrolyte (including oligomer electrolyte) type, and dimethylglyoxime (DG). Each of these chelating agents may be in the form of a free acid or a salt such as the sodium, potassium or ammonium salt. The chelating agent may also be in the form of a hydrolyzable ester derivative.

Specific examples of the aminopolycarboxylic acid type chelating agents include:
a) compounds represented by the chemical formula, R⁵⁶(Y¹)₂;
b) compounds represented by the chemical formula, N(Y¹)₃;
c) compounds represented by the chemical formula, R⁵⁶-N(Y¹)-CH₂CH₂-N(Y¹)-R⁵⁶;
d) compounds represented by the chemical formula, R⁵⁶-N(Y¹)-CH₂CH₂-N(Y¹)₂;
e) compounds represented by the chemical formula, (Y¹)₂N-R⁵⁷-N(Y¹)₂; and
f) compounds similar to the compounds of e), which contain Y¹ more than 4 groups, for example, a compound represented 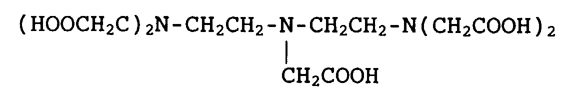 by the formula:

In the above formulae, Y¹ is -CH₂COOH or -CH₂CH₂COOH, R⁵⁶ is a group making up a known chelating agent, such as a hydrogen atom, or an alkyl, hydroxyl or hydroxyalkyl group, and R⁵⁷ is a group making up a known chelating agent of this kind, such as an alkylene or cycloalkylene group.

Typical examples of the aminopolycarboxylic acid type chelating agents include ethylenediaminetetraacetic acid (EDTA), cyclohexanediaminetetraacetic acid (CDTA), nitrilotriacetic acid (NTA), iminodiacetic acid (IDA), N-(2-hydroxyethyl)iminodiacetic acid (HIMDA), diethylenetriaminepentaacetic acid (DTPA), N-(2-hydroxyethyl)-ethylenediaminetriacetic acid (EDTA-OH) and glycol ether diaminetetraacetic acid (GEDTA) as well as salts thereof.

Examples of the aromatic and aliphatic carboxylic acid type chelating agents used in the present invention include oxalic acid, malonic acid, succinic acid, glutaric acid, adipic acid, itaconic acid, aconitic acid, pyruvic acid, salicylic acid, acetylsalicylic acid, hydroxybenzoic acid, aminobenzoic acid (including anthranilic acid). phthalic acid, trimellitic acid and gallic acid as well as salts, methyl esters and ethyl esters thereof. Examples of the amino acid type chelating agents used in the present invention include glycine, serine, alanine, lysine, cystine, cysteine, ethionine, tyrosine, methionine, and salts and derivatives thereof.

Examples of the ether polycarboxylic acid type chelating agents used in the present invention include diglycolic acid, compounds represented by the following formula: 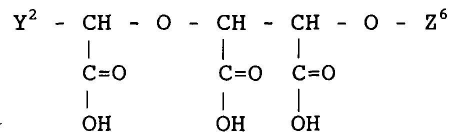 wherein Y² is a hydrogen atom, -CH₂COOH or -COOH, and Z⁶ is a hydrogen atom, -CH₂COOH or 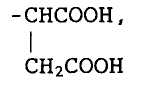 compounds similar to such compounds, and salts thereof (for example, the sodium salts).

Examples of the hydroxycarboxylic acid type chelating agents used in the present invention include malic acid, citric acid, glycolic acid, gluconic acid, heptonic acid, tartaric acid, lactic acid, and salts thereof. Examples of the phosphoric acid type chelating agents used in the present invention include orthophosphoric acid, pyrophosphoric acid, triphosphoric acid and polyphosphoric acid. Examples of the chelating agents of the polyelectrolyte (including oligomer electrolyte) type used in the present invention include acrylic acid polymers, maleic anhydride polymers, α-hydroxyacrylic acid polymers, itaconic acid polymers, copolymers composed of at least two monomers of these polymers, and epoxysuccinic acid polymers. Further, ascorbic acid, thioglycolic acid, phytic acid, glyoxylic acid and glyoxalic acid as well as salts thereof may also be suitably used as the chelating agents in the present invention.

Preferable examples of the chelating agents include ethylenediaminetetraacetic acid (EDTA), succinic acid, salicylic acid, oxalic acid, lactic acid, fumaric acid, tartaric acid, 1-hydroxyethane-1,1-diphosphonic acid and salts thereof.

The metal chelating agent of the component (E) is incorporated in a proportion of 0.1-10%, preferably 0.2-5% based on the total weight of the composition.

In the detergent compositions according to the present invention, anionic surfactants commonly used in the classical detergent compositions may be incorporated so far as no detrimental influence is thereby imposed on the effects of the present invention. Examples of such anionic surfactants include acylated isethionates, alkylsulfonates, sulfosuccinates, α-sulfo fatty acid esters, α-olefin sulfonates, phosphoric monoester type surfactants, alkyl ether carboxylates, and acylated amino acids.

Further, silicone derivatives can be incorporated in a proportion of 0.1-5% in the detergent compositions according to the present invention to give users a good feeling of sliding or dryness. No particular limitation is imposed on the silicone derivatives so far as they are those commonly used in the classical detergents and cosmetics. Examples thereof include dimethyl polysiloxane, methylphenyl polysiloxane, polyether-modified silicones, epoxy-modified silicones, alkoxy-modified silicones, fatty acid-modified silicones and fluorine-modified silicones.

When the detergent composition according to the present invention is provided as a hair shampoo, an antidandruff agent may be incorporated in a proportion of 0.1-5% in the composition. Examples of the antidandruff agent include zinc pyrithione, piroctone olamine and selenium disulfide.

In the detergent compositions according to the present invention, ingredients commonly incorporated in the classical cosmetics, drugs, food and the like, for example, germicides other than the cationic germicides, antiphlogistics such as glycyrrhetinic acid, dihydrocholesterin and allantoin, medicinally-effective agents and antiseptics; moisturizers such as propylene glycol, glycerol, diethylene glycol monoethyl ether, sorbitol and panthenol; colorants such as dyes and pigments; pearl-like-hue-imparting agents; chitosan derivatives such as hydroxypropylchitosan; various kinds of perfume bases; and besides ingredients described in ENCYCLOPEDIA OF SHAMPOO INGREDIENTS (MICELLE PRESS, 1985), may be incorporated in addition to the above-described components, as needed, so far as no detrimental influence is thereby imposed on the effects of the present invention.

The detergent compositions according to the present invention can be prepared in accordance with a method known *per se* in the art into various forms such as paste, gel, liquid and solid. The detergent compositions are suitable for use as human hair or body shampoos and can be used for animals such as horses.

The present invention will hereinafter be described in more detail by the following examples. However, the present invention is not limited to these examples. Incidentally, the amounts of individual components to be incorporated in the following examples were expressed on the basis of active ingredients.

### Examples 1 to 6 and Comparative Examples 1 to 3:

Detergent compositions of their corresponding formulations shown in Table 1 were prepared in a method known *per se* in the art to evaluate them as to detergency, irritativeness, a dry and stiff feeling after use, deodorant effect and antibacterial activity. The results are shown in Table 1.

### (1) Detergency:

Panelists composed of five men and five women were got to cleanse their bodies with each of the detergent compositions, thereby organoleptically evaluating the composition as to the detergency in accordance with the following standard.

### (Evaluation standard)

- 5:: Felt that detergency was good;
- 4:: Felt that detergency was somewhat good;
- 3:: Felt that detergency was fair;
- 2:: Felt that detergency was somewhat poor; and
- 1:: Felt that detergency was poor.

The evaluation results as to the detergency were expressed in terms of the average value of the ten panelists in accordance with the following standard:
- ⓞ:: Average value of at least 4.0 (good);
- ○:: Average value of 3.2-3.9 (somewhat good);
- △:: Average value of 2.5-3.1 (fair); and
- X:: Average value of at most 2.4 (poor).

### (2) Dry and stiff feeling after use:

Panelists composed of ten men and ten women were got to cleanse their faces with each of the detergent compositions using hot water, thereby evaluating the composition as to the dry and stiff feeling toward the face after toweling the face dry in accordance with the following standard.

### (Evaluation standard)

- 3:: Markedly gave a dry and stiff feeling;
- 2:: Clearly gave a dry and stiff feeling;
- 1:: Somewhat gave a dry and stiff feeling; and
- 0:: Gave no dry and stiff feeling.

The evaluation results as to the dry and stiff feeling were expressed in terms of the average value of the twenty panelists in accordance with the following standard:
- ⓞ:: Average value smaller than 0.5;
- ○:: Average value not smaller than 0.5, but smaller than 1.5;
- △:: Average value not smaller than 1.5, but smaller than 2.5; and
- X:: Average value not smaller 2.5.

### (3) Irritativeness:

The upper arms of panelists composed of five men and five women were treated with a ten-fold diluted solution of each of the detergent compositions shown in Table 1 twice a day for two weeks under conditions of contact time of 5 minutes by a cup shaking method.

The irritativeness was evaluated by observing the condition of the skin by an expert valuer upon elapsed time of 2 hours after the final treatment, and ranked in accordance with the following standard.

### (Evaluation standard)

- 4:: Erythema was observed;
- 3:: Desquamation was observed;
- 2:: Desquamation was slightly observed;
- 1:: Desquamation was scarcely observed;
- 0:: No skin change was observed.

The evaluation results as to the irritativeness were expressed in terms of the average value of the ten panelists in accordance with the following standard:
- ⓞ:: Average value not greater than 1.0 (not irritative);
- ○:: Average value of 1.1-2.0 (scarcely irritative);
- △:: Average value of 2.1-3.0 (slightly irritative); and
- X:: Average value not smaller 3.1 (irritative).

### (4) Deodorant effect:

With respect to each of the detergent compositions, a service test was conducted once a day for 2 weeks with panelists composed of five men and five women.

The deodorant effect was evaluated by an expert valuer as to whether the panelists had a body smell or not upon elapsed time of 24 hours after the final cleansing and ranked in accordance with the following standard.

### (Evaluation standard)

- 5:: Not smelled;
- 4:: Slightly smelled;
- 3:: Smelled;
- 2:: Considerably smelled; and
- 1:: Strongly smelled.

The evaluation results as to the deodorant effect were expressed in terms of the average value of the ten panelists in accordance with the following standard:
- ⓞ:: At least 4.0 (good);
- ○:: 3.2-3.9 (somewhat good);
- △:: 2.5-3.1 (fair); and
- X:: At most 2.4 (poor).

### (5) Antibacterial activity:

### Method of antibacterial test:

Germs, *Staphylococcus aureus* IFO 12732 (St. aureus) and *Escherichia coli* IFO 3972 (E. coli) to be tested were separately precultured at 35°C using an SCD medium (product of Nippon Seiyaku K.K.).

Each of the culture solutions of St. aureus and E. coli precultured was spread on an SCD agar medium (product of Nippon Seiyaku K.K.). After 50 µl of each of the detergent compositions shown in Table 1 were poured into the agar medium, a paper disk (diameter: 8 mm) washed with water was placed on the agar medium, on which the culture solution had been spread, to measure a diameter (mm) of an inhibitory zone after 24 hours at 35°C, thereby judging the antibacterial activity.

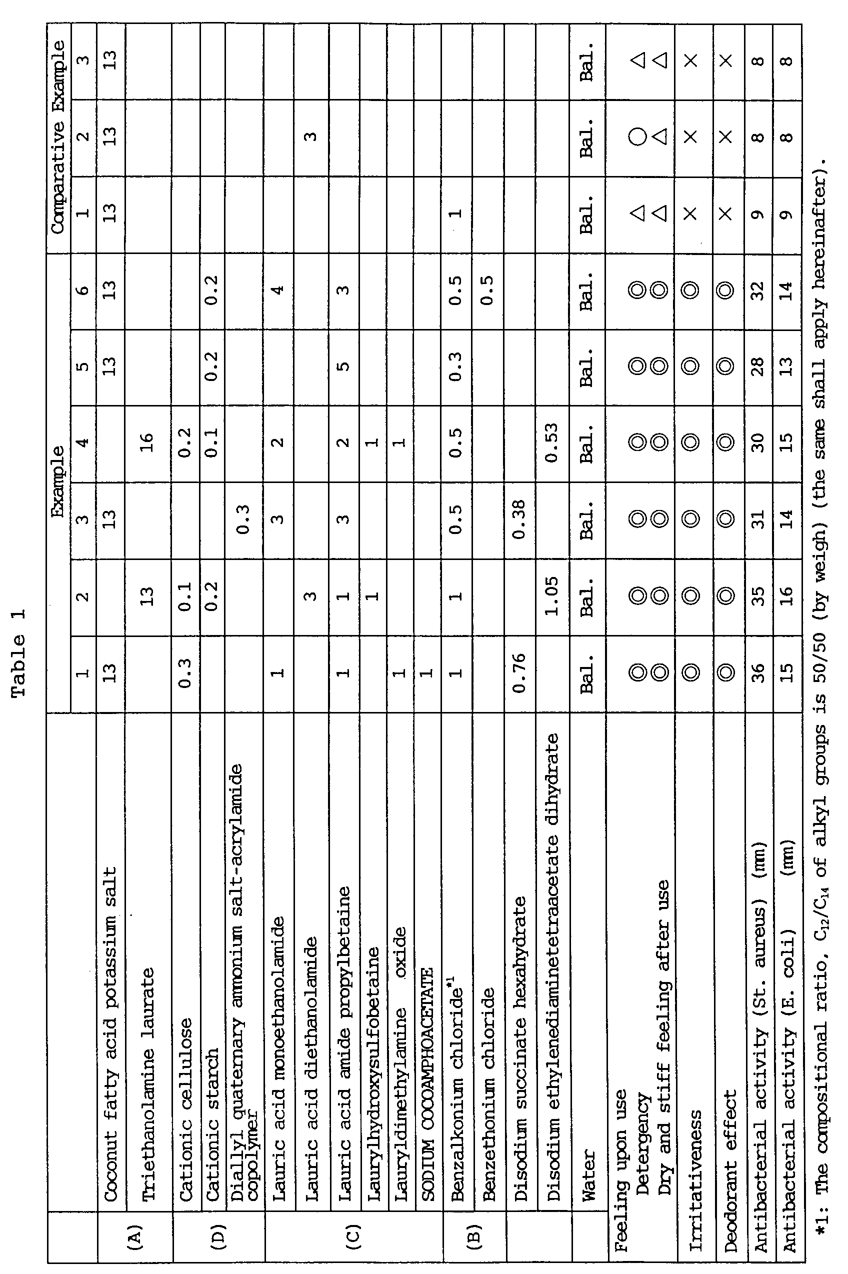

### Example 7:

A hand soap of a formulation shown below was produced in accordance with a method known *per se* in the art.

| | |
|---|---|
| Coconut fatty acid potassium salt | 16% |
| Cationic cellulose (Polymer JR400) | 0.3% |
| Lauric acid diethanolamide | 3% |
| Benzalkonium chloride^{*1} | 1% |
| Disodium ethylenediaminetetraacetate | 0.6% |
| Glycerol | 1% |
| Methylparaben | 0.2% |
| Perfume base | 0.1% |
| Water | Balance |
| Total | 100.0% |

| | |
|---|---|
| *1: The compositional ratio, C₁₂/C₁₄ of alkyl groups is 50/50 ( by weigh) (the same shall apply hereinafter). | |

The hand soap thus obtained had excellent detergent performance and foamability and low irritativeness, gave users no dry and stiff feeling toward the skin and had a germicidal effect.

### Example 8:

A body shampoo of a formulation shown below was produced in accordance with a method known *per se* in the art.

| | |
|---|---|
| Coconut fatty acid potassium salt | 30% |
| Cationic starch | 0.5% |
| Lauric acid amide propylbetaine | 2% |
| Lauryldimethylamine oxide | 0.5% |
| SODIUM COCOAMPHOACETATE | 0.3% |
| Benzalkonium chloride^{*1} | 1% |
| Disodium succinate | 0.8% |
| Glycerol | 1% |
| Magnesium stearate | 0.5% |
| Pearl-like-hue-imparting agent (ethylene glycol distearate) | 1% |
| Methylparaben | 0.2% |
| Perfume base | 0.2% |
| Water | Balance |
| Total | 100.0% |

| | |
|---|---|
| *1: The compositional ratio, C₁₂/C₁₄ of alkyl groups is 50/50 ( by weigh) (the same shall apply hereinafter). | |

The body shampoo thus obtained had excellent detergent performance and foamability and low irritativeness, gave users no dry and stiff feeling toward the skin after use and had a deodorant effect.

### Example 9:

A solid body soap of a formulation shown below was produced in accordance with a method known *per se* in the art.

| | |
|---|---|
| Soap base^{*2} | Balance |
| Cationic cellulose (Polymer JR400) | 0.5% |
| Lauric acid amide propylbetaine | 2.0% |
| Benzethonium chloride | 1% |
| Disodium ethylenediaminetetraacetate | 1% |
| 1-Hydroxyethane-1,1-diphosphonic acid solution (60% aqueous solution) | 0.4% |
| Dibutylhydroxytoluene | 0.1% |
| Magnesium stearate | 0.5% |
| Sodium benzoate | 0.5% |
| Methylparaben | 0.2% |
| Titanium oxide | 0.2% |
| Perfume base | 0.5% |
| Total | 100.0% |

| | |
|---|---|
| *2: Sodium salt of a mixed fatty acid composed of beef tallow/coconut oil = 80/20 (by weight); water content: 13%. | |

The solid body soap thus obtained had excellent detergent performance and foamability and low irritativeness, gave users no dry and stiff feeling toward the skin after use and had a deodorant effect.

### Examples 10 to 17 and Comparative Examples A to D:

Detergent compositions of their corresponding formulations shown in Table 2 were prepared in a method known *per se* in the art to evaluate them as to detergency, a feeling after use, antidandruffy effect and deodorant effect. The results are shown in Table 2.

### 〈Evaluation method and standard of detergency〉

Healthy five men and five women were got to shampoo their hair with each of the detergent compositions once a day for a week, thereby evaluating the composition as to the detergency in accordance with the following standard.

### (Evaluation standard)

- 5:: Felt that detergency was good;
- 4:: Felt that detergency was somewhat good;
- 3:: Felt that detergency was fair;
- 2:: Felt that detergency was somewhat poor; and
- 1:: Felt that detergency was poor.

The evaluation results as to the detergency were expressed in terms of the average value of the ten panelists in accordance with the following standard:
- ⓞ:: At least 4.0, good detergency;
- ○:: 3.2-3.9, somewhat good detergency;
- △:: 2.5-3.1, fair detergency; and
- X:: At most 2.4, poor detergency.

### 〈Evaluation method and standard of moisturized feeling after shampooing〉

Healthy ten women were got to shampoo their hair with each of the detergent compositions for a week, thereby organoleptically evaluating the composition as to the feeling toward the hair after the shampooing in accordance with the following standard.

### (Evaluation standard)

- 4:: Felt that the hair became moisturized after shampooing;
- 3:: Felt that the hair became somewhat moisturized after shampooing;
- 2:: Felt that the hair became somewhat dry after shampooing; and
- 1:: Felt that the hair became dry after shampooing.

The evaluation results as to the moisturized feeling were expressed in terms of the average value of the ten panelists in accordance with the following standard:
- ⓞ:: At least 4.0, good feeling;
- ○:: 3.2-3.9, somewhat good feeling;
- △:: 2.5-3.1, fair feeling; and
- X:: At most 2.4, poor feeling.

### 〈Evaluation method and standard of antidandruffy effect〉

Monitors composed of five men were got to shampoo their hair with each of the detergent compositions once a day for a month. After the final shampooing, shampooing was stopped for about 48 hours, and the hair was shampooed again twice with each 3 g of the detergent composition, thereby collecting all the washings.

The whole amount of the washings thus collected was filtered through a 50 nylon mesh to remove unnecessary dust and hair. The whole amount of the filtrate was filtered through a 255 nylon mesh (100 x 100 µm) weighed in advance. After the nylon mesh was air-dried at room temperature for about 48 hours, the weight of the horny layer captured on the 255 nylon mesh was regarded as the weight of dandruff.

The evaluation results as to the antidandruffy effect were expressed in terms of the average value of the weight of dandruff of the five men in accordance with the following standard:
- ⓞ:: Weight of dandruff ≤ 30 mg;
- ○:: 30 mg < Weight of dandruff ≤ 40 mg;
- △:: 40 mg < Weight of dandruff ≤ 50 mg; and
- X:: 50 mg < Weight of dandruff.

### 〈Evaluation method and standard of deodorant effect〉

With respect to each of the detergent compositions, a service test was conducted once a day for 2 weeks with panelists composed of five men and five women.

The deodorant effect was evaluated by an expert valuer as to whether the panelists had a body smell or not upon elapsed time of 24 hours after the final cleansing and ranked in accordance with the following standard.

### (Evaluation standard)

- 5:: Not smelled;
- 4:: Slightly smelled;
- 3:: Smelled;
- 2:: Considerably smelled; and
- 1:: Strongly smelled.

The evaluation results as to the deodorant effect were expressed in terms of the average value of the ten panelists in accordance with the following standard:
- ⓞ:: At least 4.0, good deodorant effect;
- ○:: 3.2-3.9, somewhat good deodorant effect;
- △:: 2.5-3.1, fair deodorant effect; and
- X:: At most 2.4, poor deodorant effect.

### 〈Testing method of antibacterial activity〉

Germs, *Staphylococcus aureus* IFO 12732 (St. aureus) and *Malass ezia furfur* IFO 658 (M. furfur) to be tested were precultured at 35°C using an SCD medium (product of Nippon Seiyaku K.K.) and a Malt-Yeast medium [containing 0.5% of Casitone (product of Difco Co.), 0.67% of yeast extract (product of Difco Co.), 0.5% yeast-nitrogen-base (product of Difco Co.), 2% of glucose and 0.1% of Rheodol TW-S120 (product of Kao Corporation)], respectively.

The culture solutions of St. aureus and M. furfur precultured were spread on an SCD agar medium (product of Nippon Seiyaku K.K.) and a Malt agar medium [containing 2% of Malt extract (product of Difco Co.), 0.1% of Rheodol TW-S120 (product of Kao Corporation) and 1.5% of agar], respectively. After 50 µl of each of the detergent compositions shown in Table 2 were poured into the agar media, a paper disk (diameter: 8 mm) washed with water was placed on the agar media, on which the respective culture solutions had been spread, to measure a diameter (mm) of an inhibitory circle after 24 hours at 35°C, thereby judging the antibacterial activity.

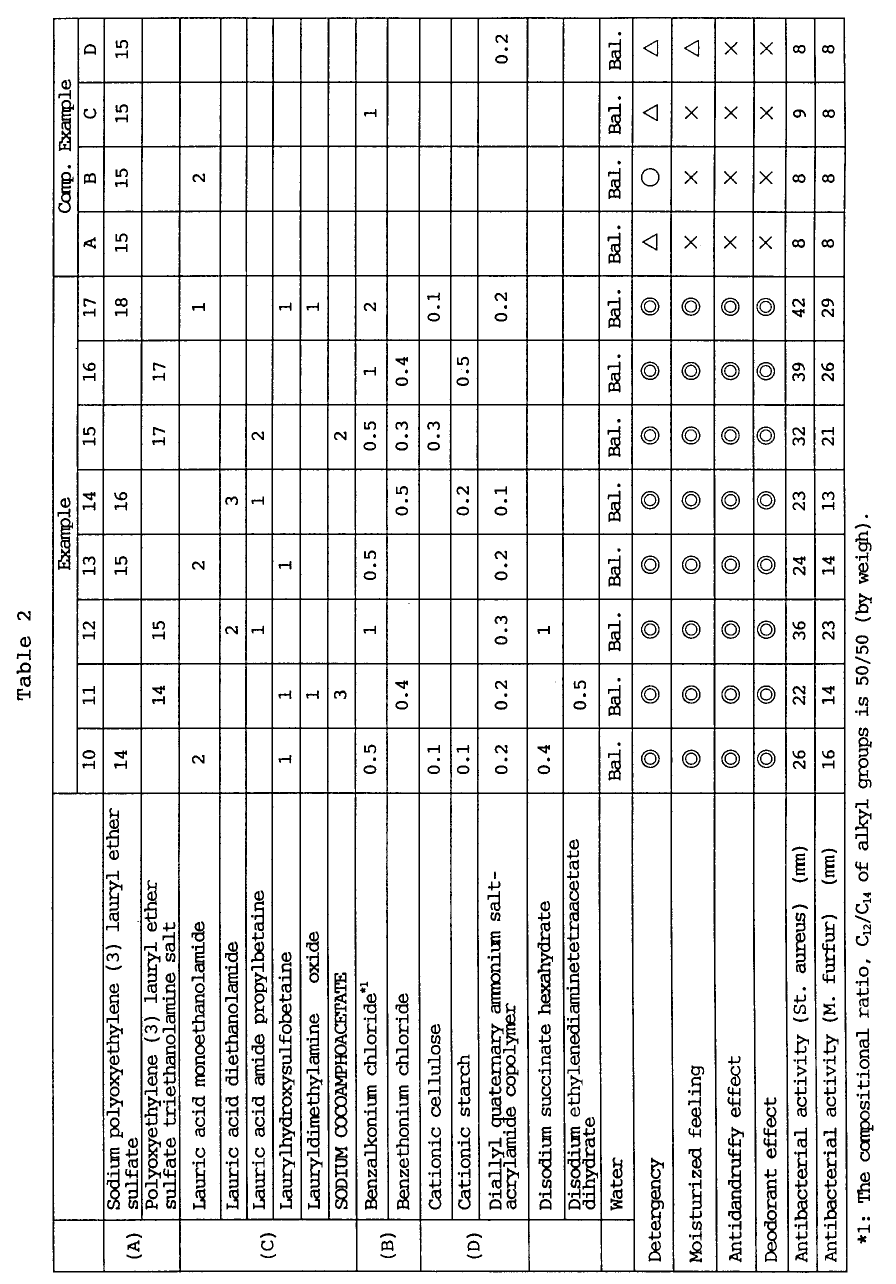

### Example 18:

A head and body shampoo of a formulation shown below was produced in accordance with a method known *per se* in the art.

| | |
|---|---|
| Sodium polyoxyethylene cocoyl ether sulfate (EO = 3) | 16% |
| Coconut fatty acid diethanolamide | 1% |
| Lauric acid amide propylbetaine | 1% |
| SODIUM COCOAMPHOACETATE | 2% |
| Cetyl pyridinium chloride | 1% |
| Cationic starch | 0.5% |
| Disodium ethylenediaminetetraacetate | 1% |
| Methylparaben | 0.2% |
| Pearl-like-hue-imparting agent (ethylene glycol distearate) | 1% |
| Perfume base | 0.1% |
| Water | Balance |
| Total | 100.0% |

The head and body shampoo thus obtained had excellent detergency, gave users a pleasant feeling after use and had deodorant and antidandruffy effects.

### Example 19:

A hand soap of a formulation shown below was produced in accordance with a method known *per se* in the art.

| | |
|---|---|
| Sodium polyoxyethylene lauryl ether sulfate (EO = 2) | 18% |
| SODIUM COCOAMPHOACETATE | 3% |
| Lauric acid diethanolamide | 1% |
| Benzalkonium chloride^{*1} | 0.8% |
| Cationic cellulose | 0.2% |
| Disodium succinate | 0.6% |
| 1-Hydroxyethane-1,1-diphosphonic acid solution (60% aqueous solution) | 0.2% |
| Butylparaben | 0.2% |
| Pearl-like-hue-imparting agent (ethylene glycol distearate) | 2% |
| Perfume base | 0.2% |
| Water | Balance |
| Total | 100.0% |

| | |
|---|---|
| *1: The compositional ratio, C₁₂/C₁₄ of alkyl groups is 50/50 (by weigh). | |

The hand soap thus obtained had excellent detergency, gave users a pleasant feeling after use and had germicidal and antibacterial effects.

### Example 20:

A hair shampoo of a formulation shown below was produced in accordance with a method known *per se* in the art.

| | |
|---|---|
| Ammonium polyoxyethylene lauryl ether sulfate (EO = 3) | 20% |
| Lauric acid amide propylbetaine | 2% |
| SODIUM LAUROAMPHOACETATE | 2% |
| Benzalkonium chloride^{*1} | 1% |
| Cationic cellulose | 0.5% |
| Disodium succinate | 0.8% |
| Propylene glycol | 0.5% |
| Glycerol | 1% |
| Butylparaben | 0.2% |
| Perfume base | 0.3% |
| Water | Balance |
| Total | 100.0% |

| | |
|---|---|
| *1: The compositional ratio, C₁₂/C₁₄ of alkyl groups is 50/50 (by weigh). | |

The hair shampoo thus obtained had excellent detergency, gave users a pleasant feeling toward the hair after use and had an antidandruffy effect.

## Claims

1. A detergent composition comprising the following components (A), (B), (C) and (D):
(A) 5-95 wt.% of at least one anionic surfactant selected from the group consisting of higher fatty acid salts represented by the general formula (1):
R¹COOM¹ (1)
wherein R¹ is a linear or branched alkyl or alkenyl group having 7-21 carbon atoms, and M¹ is an alkali metal or alkaline earth metal atom, or an ammonium, alkylammonium or alkanolammonium group, and sulfuric ester type surfactants represented by the general formula (2):
R²O-(CH₂CH₂O)ₙ-SO₃M² (2)
wherein R² is a linear or branched alkyl or alkenyl group having 8-20 carbon atoms, n is a number of 0-10 on the average, and M² is an alkali metal or alkaline earth metal atom, or an ammonium, alkylammonium or alkanolammonium group;
(B) 0.2-5 wt.% of at least one selected from the group consisting of cationic germicides represented by the following general formulae (3), (4), (5) and (6): 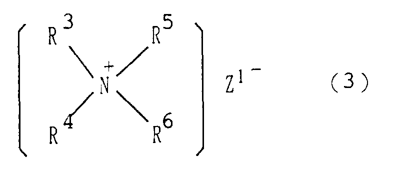 wherein R³ and R⁴ may be the same or different from each other and are independently a long-chain alkyl, long-chain alkenyl or long-chain hydroxyalkyl group having 6-14 carbon atoms, said groups R³ and R⁴ having 16-26 carbon atoms in total, R⁵ and R⁶ may be the same or different from each other and are independently an alkyl or hydroxyalkyl group having 1-3 carbon atoms, or a polyoxyethylene group having an average number of moles of at most 10, and Z¹ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin; 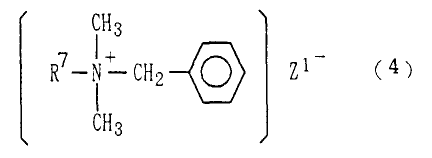 wherein R⁷ is a hydrocarbon group having 8-14 carbon atoms or a group represented by the formula: 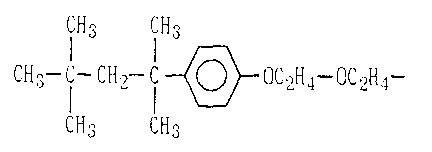 and Z¹ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerisation degree of at least 3 or containing a condensate of a sulfonated polycyclic aromatic compound, which may have a hydrocarbon group as a substituent group, with formalin; 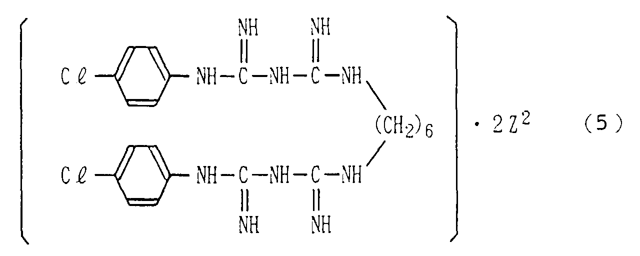 wherein Z² is gluconic acid, acetic acid or hydrochloric acid; and 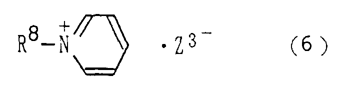 wherein R⁸ is a linear or branched alkyl group having 6-18 carbon atoms, Z³ is a halogen atom, an anionic residue of an amino acid, a fatty acid, or a phosphate, phosphonate, sulfonate or sulfate having a linear or branched alkyl or alkenyl group having 1-30 carbon atoms, or an anionic oligomer or polymer having a styrenesulfonic acid having a polymerization degree of at least 3 or containing a condensate of a sulfonated polycyclic aromatic compound, which may have a hydrocarbon group as a substituent group, with formalin, and polyhexamethylene biguanide;
(C) 0.1-10 wt.% of at least one selected from the group consisting of amphoteric surfactants, amine oxide type surfactants, alkanolamide type surfactants and amidoamino acid type surfactants; and
(D) 0.01-5 wt.% of a cationic polymer, wherein the component (A) is contained as a principal detergent base.

2. The detergent composition according to Claim 1, wherein the content of the component (A) is 5-50 wt.% based on the total weight of the composition, and the composition is in the form of liquid.

3. The detergent composition according to Claim 1, wherein the content of the component (A) is 10-80 wt.% based on the total weight of the composition, and the composition is in the form of paste.

4. The detergent composition according to Claim 1, wherein the content of the component (A) is 60-95 wt.% based on the total weight of the composition, and the composition is in the form of solid.

5. The detergent composition according to any one of Claims 1 to 4, which further comprises at least one metal chelating agent.
